# EUROPEAN PATENT APPLICATION

(11) **EP 3 922 708 A1**
(43) Date of publication of application: **15.12.2021**
(21) Application number: 19914235.7
(22) Date of filing: 10.12.2019
(51) Int. Cl.: C12M 1/00, G01N 27/00

(54) **CELLULAR POTENTIAL MEASUREMENT SUBSTRATE, METHOD FOR PRODUCING SAME, AND CELL CULTURE SUBSTRATE**

(30) Priority: 08.02.2019 JP 2019021596; 27.11.2019 JP 2019214533
(71) Applicant: Panasonic Intellectual Property Management Co., Ltd., Osaka-shi, Osaka 540-6207 (JP)
(72) Inventor: TSUJI, Kiyotaka, Osaka 540-6207 (JP)
(74) Representative: Grünecker Patent- und Rechtsanwälte PartG mbB
(86) International application number: PCT/JP2019/048206
(87) International publication number: WO 2020/162031

(57) **Abstract**

The present disclosure provides a cellular potential measurement substrate capable of reducing entering of an adhesive into a culture region, the adhesive being used for bonding a frame plate that defines the culture region to an insulating substrate. A cellular potential measurement substrate (100) according the present disclosure includes an insulating substrate (110) having, on a main surface (111) thereof, a culture region (112) for culturing a cell, a measurement electrode (120) disposed in the culture region (110) and configured to measure a potential of the cell, a plurality of insulating fibers (130) arranged on the culture region (110) side by side at intervals in a Y-direction and each extending in an X-direction, a frame plate (140) disposed on the plurality of insulating fibers (130) and having a culture hole (141) at a position corresponding to the culture region (110), and an adhesive (150) that has bonded the frame plate (140) to the insulating substrate (110). The frame plate (140) further has a first adhesion through-hole (142) into which the adhesive (150) has been injected. The first adhesion through-hole (142) is disposed in a first region (146) that does not overlap the culture hole (141) when viewed in the first direction.

## Description

### Technical Field

The present disclosure relates to a cellular potential measurement substrate that measures a potential of a cell, a method for producing the same, and a cell culture substrate.

### Background Art

There have been proposed substrates that culture cells in regions in which electrodes are formed and that measure the potentials of the cultured cells by using the electrodes. For example, Patent Literature 1 discloses a cellular potential measurement electrode assembly including an insulating substrate, a measurement electrode that is in contact with a conductive pattern disposed in the insulating substrate, and insulating fibers disposed on the insulating substrate.

In Patent Literature 2, a silicone resin sheet that covers a region excluding a periphery of electrodes (that is, a culture region) is bonded to a substrate with a silicone adhesive.

### Citation List

### Patent Literature

PTL 1: Japanese Unexamined Patent Application Publication No. 2016-212083
PTL 2: Japanese Unexamined Patent Application Publication No. 2018-143144

### Summary of Invention

### Technical Problem

As in Patent Literature 2, when a silicone resin sheet (a frame plate in the present disclosure) is bonded to an insulating substrate with an adhesive, the adhesive may enter the culture region along insulating fibers. As a result, the insulating fibers and measurement electrodes in the culture region are covered with the adhesive, which may result in inhibition of cell culture and interference of potential measurement.

In view of this, the present disclosure provides a cellular potential measurement substrate and the like capable of reducing entering of an adhesive into a culture region, the adhesive being used for bonding a frame plate that defines the culture region to a substrate.

### Solution to Problem

A cellular potential measurement substrate according to an aspect of the present disclosure includes an insulating substrate having, on a main surface thereof, a culture region for culturing a cell, a measurement electrode disposed in the culture region and configured to measure a potential of the cell, a plurality of insulating fibers arranged on the culture region side by side at intervals in a second direction and each extending on the main surface in a first direction perpendicular to the second direction, a frame plate disposed on the plurality of insulating fibers and having a culture hole at a position corresponding to the culture region, and an adhesive that has bonded the frame plate to the insulating substrate. The frame plate further has a first adhesion through-hole into which the adhesive has been injected. The first adhesion through-hole is disposed in a first region that does not overlap the culture hole when viewed in the first direction.

A method for producing a cellular potential measurement substrate according to an aspect of the present disclosure includes a step of arranging, on an insulating substrate having a culture region in which a measurement electrode is disposed, a plurality of insulating fibers side by side at intervals in a second direction, the insulating fibers each extending on the main surface in a first direction perpendicular to the second direction, a step of disposing, on the insulating substrate, a frame plate having a culture hole such that a position of the culture hole and a position of the culture region are aligned with each other, and a step of injecting an adhesive into a first adhesion through-hole formed in a first region of the frame plate, the first region not overlapping the culture hole when viewed in the first direction.

A cell culture substrate according to an aspect of the present disclosure includes a substrate having, on a main surface thereof, a culture region for culturing a cell, a plurality of fibers arranged on the culture region side by side at intervals in a second direction and each extending on the main surface in a first direction perpendicular to the second direction, a frame plate disposed on the plurality of fibers and having a culture hole at a position corresponding to the culture region, and an adhesive that has bonded the frame plate to the substrate. The frame plate further has a first adhesion through-hole into which the adhesive has been injected. The first adhesion through-hole is disposed in a first region that does not overlap the culture hole when viewed in the first direction. Advantageous Effects of Invention

The cellular potential measurement substrate and the like according to the present disclosure are capable of reducing entering of an adhesive into a culture region, the adhesive being used for bonding a frame plate that defines the culture region to a substrate.

### Brief Description of Drawings

[Fig. 1] Fig. 1 is a perspective view of a cellular potential measurement substrate according to Embodiment 1.
[Fig. 2] Fig. 2 is an exploded perspective view of the cellular potential measurement substrate according to Embodiment 1.
[Fig. 3] Fig. 3 is a plan view of the cellular potential measurement substrate according to Embodiment 1.
[Fig. 4] Fig. 4 is an enlarged plan view of a frame plate portion of the cellular potential measurement substrate according to Embodiment 1.
[Fig. 5] Fig. 5 is a flowchart illustrating a method for producing the cellular potential measurement substrate according to Embodiment 1.
[Fig. 6] Fig. 6 is an enlarged plan view of a frame plate portion of a cellular potential measurement substrate according to Modification 1 of Embodiment 1.
[Fig. 7] Fig. 7 is an enlarged plan view of a frame plate portion of a cellular potential measurement substrate according to Modification 2 of Embodiment 1.
[Fig. 8] Fig. 8 is a plan view of a cellular potential measurement substrate according to Embodiment 2.
[Fig. 9] Fig. 9 is an enlarged plan view of the cellular potential measurement substrate according to Embodiment 2.
[Fig. 10] Fig. 10 is a plan view of a measurement electrode in Embodiment 2.
[Fig. 11] Fig. 11 is a sectional view of the measurement electrode in Embodiment 2.
[Fig. 12] Fig. 12 is an enlarged plan view of a frame plate of the cellular potential measurement substrate according to Embodiment 2.
[Fig. 13] Fig. 13 is an enlarged plan view of a frame plate of a cellular potential measurement substrate according to Modification 1 of Embodiment 2.
[Fig. 14] Fig. 14 is an enlarged plan view of a frame plate of a cellular potential measurement substrate according to Modification 2 of Embodiment 2.
[Fig. 15] Fig. 15 is an enlarged plan view of a frame plate of a cellular potential measurement substrate according to Modification 3 of Embodiment 2.
[Fig. 16] Fig. 16 is an enlarged plan view of insulating fibers according to another embodiment.
[Fig. 17] Fig. 17 is a perspective view of a cell culture substrate according to another embodiment.

### Description of Embodiments

Embodiments will be specifically described below with reference to the attached drawings.

Each of the embodiments described below indicates a general or specific example. Numerical values, shapes, materials, components, arrangement positions and connection forms of the components, steps, the order of the steps, and the like described in the embodiments below are only exemplary and are not intended to limit the scope of the claims. Among the components in the following embodiments, components that are not described in an independent claim indicating the broadest concept are described as optional components. The drawings are not necessarily strict illustrations. In the drawings, substantially the same components are assigned the same reference numerals, and overlapping descriptions may be omitted or simplified.

In the present disclosure, an X-direction is a first direction in which insulating fibers extend on a main surface of an insulating substrate. A Y-direction is a second direction perpendicular to the first direction on the main surface of the insulating substrate. A Z-direction is a direction perpendicular to the main surface of the insulating substrate. The expression "in plan view" means viewing the X-Y plane in the Z-direction.

In the present disclosure, terms indicating relationships between elements, such as parallel and perpendicular, terms indicating shapes of elements, such as rectangle, and numerical values are meant to include not only strict meanings but also substantially equivalent ranges with a difference of, for example, about several percent.

In the drawings of the present disclosure, broken lines indicate components that are not visible from a surface and boundaries of regions.

### (Embodiment 1)

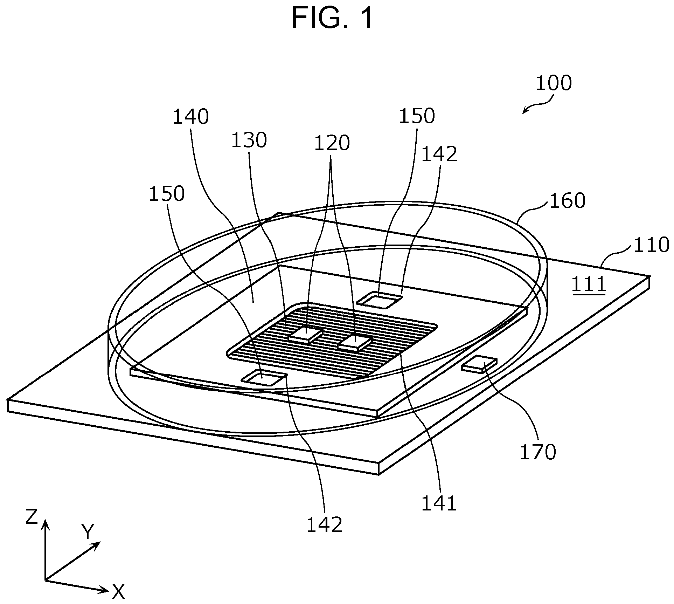

Embodiment 1 will be specifically described below with reference to Figs. 1 to 5.

### [Structure of cellular potential measurement substrate]

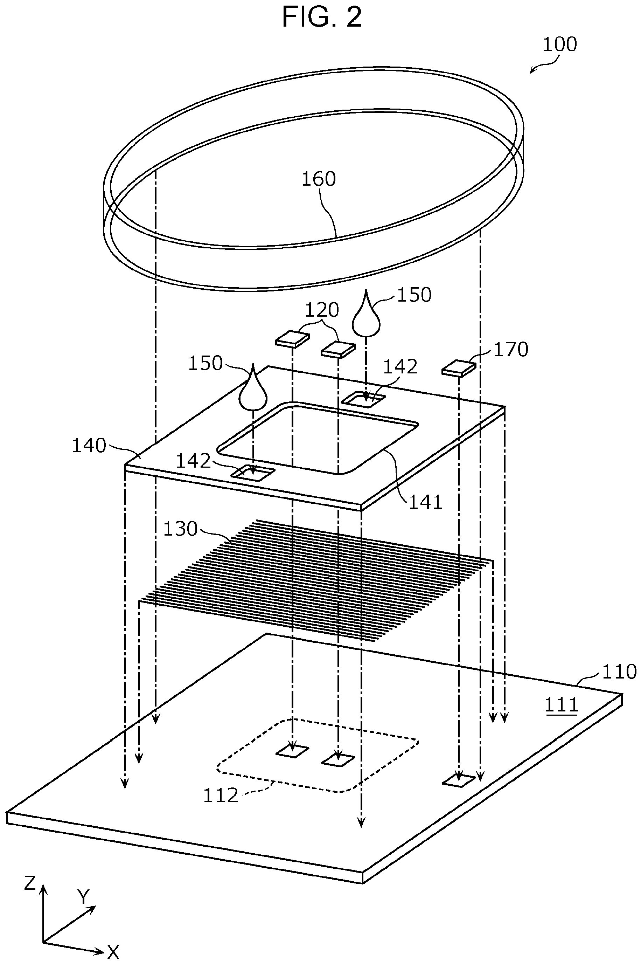

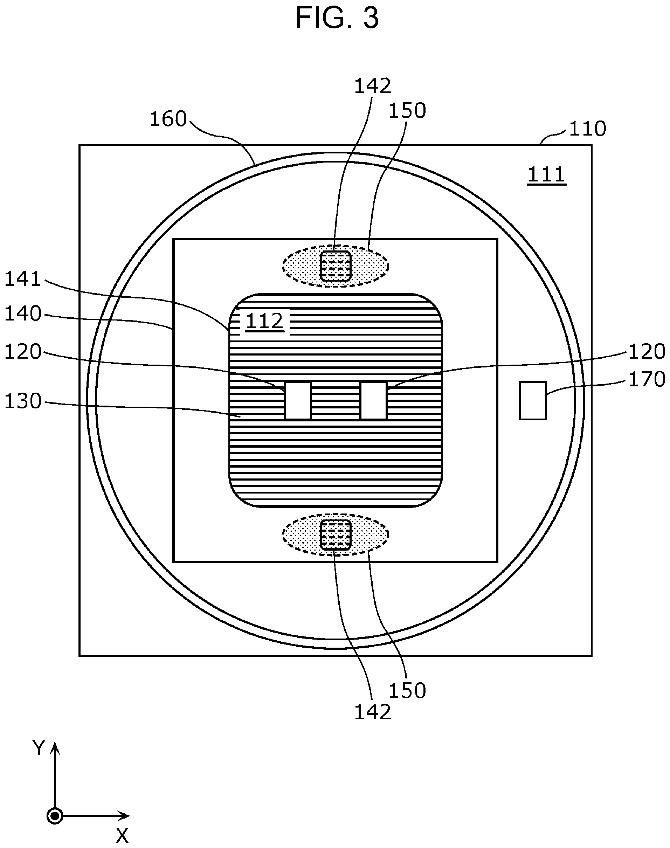

First, the structure of a cellular potential measurement substrate 100 according to the present embodiment will be described with reference to Figs. 1 to 3. Fig. 1 is a perspective view of a cellular potential measurement substrate 100 according to Embodiment 1. Fig. 2 is an exploded perspective view of the cellular potential measurement substrate 100 according to Embodiment 1. Fig. 3 is a plan view of the cellular potential measurement substrate 100 according to Embodiment 1.

The cellular potential measurement substrate 100 is a plate-like assembly configured to measure an electric potential of a cell. As illustrated in Figs. 1 to 3, the cellular potential measurement substrate 100 includes an insulating substrate 110, measurement electrodes 120, insulating fibers 130, a frame plate 140, an adhesive 150, a surrounding wall 160, and a reference electrode 170. Each of the components of the cellular potential measurement substrate 100 will be described in detail below.

The insulating substrate 110 is an insulating substrate having a main surface 111. The insulating substrate 110 has, on the main surface 111, a culture region 112 for culturing cells. In this embodiment, the culture region 112 is a region having a rectangular shape with rounded corners in plan view.

The cells to be cultured may be, for example, cardiomyocyte-like cells obtained by differentiation induction. The cells are not limited to cardiomyocyte-like cells and may be other cells.

The measurement electrodes 120 are electrodes configured to measure electric potentials of cells and are disposed in the culture region 112. In this embodiment, the number of measurement electrodes 120 is two. The size of each of the measurement electrodes 120 is about 15 micrometers × about 170 micrometers. The distance between the two measurement electrodes 120 is about 400 micrometers. The number, the size, and the distance are non-limiting examples.

The measurement electrodes 120 are connected to a potential measurement device (not shown, for example, an amplifier for measuring a cellular potential) through wiring lines (not shown) formed in the insulating substrate 110. The material of the measurement electrodes 120 may be, for example, platinum black but is not limited to this.

The insulating fibers 130 are arranged on the culture region 112 side by side at intervals in the Y-direction and each extend in the X-direction. Portions of the insulating fibers 130 are sandwiched between the frame plate 140 and the insulating substrate 110. In this embodiment, the insulating fibers 130 are formed of polymethylglutarimide, and the surface coverage of the insulating fibers 130 is about 30%. The material and the surface coverage are non-limiting examples.

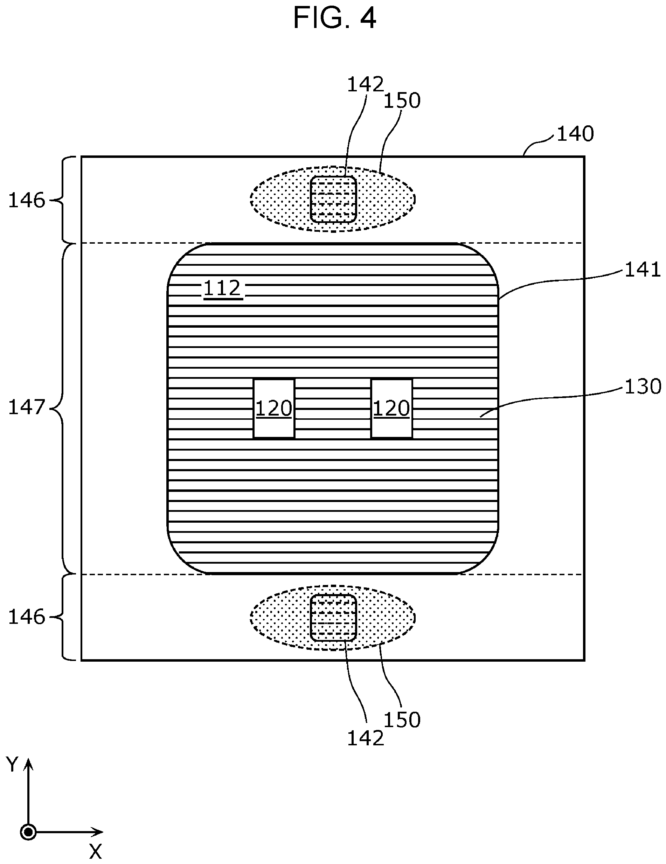

The frame plate 140 is disposed on the insulating fibers 130 and has a culture hole 141 and first adhesion through-holes 142 that are arranged to be spaced apart from each other. Herein, the frame plate 140 is referred to as a plate but may be referred to as a sheet depending on the thickness thereof. The frame plate 140 may be, for example, a silicone resin sheet having a thickness of about 1 millimeter but is not limited to this. The detailed structure of the frame plate 140 will be described later with reference to Fig. 4.

The adhesive 150 bonds the frame plate 140 to the insulating substrate 110. In the production process of the cellular potential measurement substrate 100, the adhesive 150 is injected into the first adhesion through-holes 142 and spreads in the X-direction mainly along the insulating fibers 130 between the frame plate 140 and insulating substrate 110, as illustrated in Fig. 3. That is, the adhesive 150 spreads largely in the X-direction rather than in the Y-direction. The adhesive 150 may be, for example, a silicone adhesive but is not limited to this.

The surrounding wall 160 is a wall member that surrounds the culture region 112 and the frame plate 140. The region surrounded by the surrounding wall 160 can be filled with a culture solution. The surrounding wall 160 may be a ring-shaped glass member having a height of 10 millimeters and a thickness of 1.5 millimeters but is not limited to this.

The reference electrode 170 is an electrode configured to measure an electric potential serving as a reference. The reference electrode 170 is disposed in a region outside the culture region 112, the region being included in the region surrounded by the surrounding wall 160 on the main surface 111 of the insulating substrate 110. The reference electrode 170 has an area of, for example, about 200 square micrometers. The reference electrode 170 is connected to the potential measurement device through a wiring line formed in the insulating substrate 110 as in the measurement electrodes 120. In this case, the potential measurement device can measure the potential difference between each measurement electrode 120 and the reference electrode 170. The material of the reference electrode 170 may be, for example, platinum black but is not limited to this.

The surrounding wall 160 and the reference electrode 170 are not necessarily included in the cellular potential measurement substrate 100. For example, when a culture solution is not necessary, the surrounding wall 160 may be omitted. For example, the reference electrode 170 may be inserted from the surface of the culture solution into the culture solution when measurement is performed.

### [Structure of frame plate]

Next, the structure of the frame plate 140 will be specifically described with reference to Fig. 4. Fig. 4 is an enlarged plan view of a portion of the frame plate 140 of the cellular potential measurement substrate100 according to Embodiment 1.

As described above, the frame plate 140 has a culture hole 141 and first adhesion through-holes 142.

The culture hole 141 penetrates the frame plate 140 in the Z-direction and is disposed at a position corresponding to the culture region 112. That is, the frame plate 140 defines, with the culture hole 141, the culture region 112 on the main surface 111 of the insulating substrate 110. In this embodiment, the culture hole 141 has a rectangular shape with rounded corners in plan view. The number of culture holes 141 is one.

The first adhesion through-holes 142 penetrate the frame plate 140 in the Z-direction and each have a rectangular shape with rounded corners in plan view. The number of first adhesion through-holes 142 is two.

The first adhesion through-holes 142 are each disposed in a first region 146 that does not overlap the culture hole 141 when viewed in the X-direction. That is, the first adhesion through-holes 142 are each disposed outside a second region 147 that overlaps the culture hole 141 when viewed in the X-direction. In other words, the first adhesion through-holes 142 do not overlap the culture hole 141 when viewed in the X-direction. That is, when the frame plate 140 is projected onto the Y-Z plane in the X-direction, projection regions of the first adhesion through-holes 142 do not overlap a projection region of the culture hole 141.

In this embodiment, since the culture hole 141 has a rectangular shape with rounded corners, the first regions 146 are divided from the second region 147 by sides of the culture hole 141, the sides extending in the X-direction, and extensions of the sides.

Note that, in this embodiment, the frame plate 140 has no adhesion through-hole in the second region 147. That is, the adhesion through-holes are present only in the first regions 146 and are not present in the second region 147.

### [Method for producing cellular potential measurement substrate]

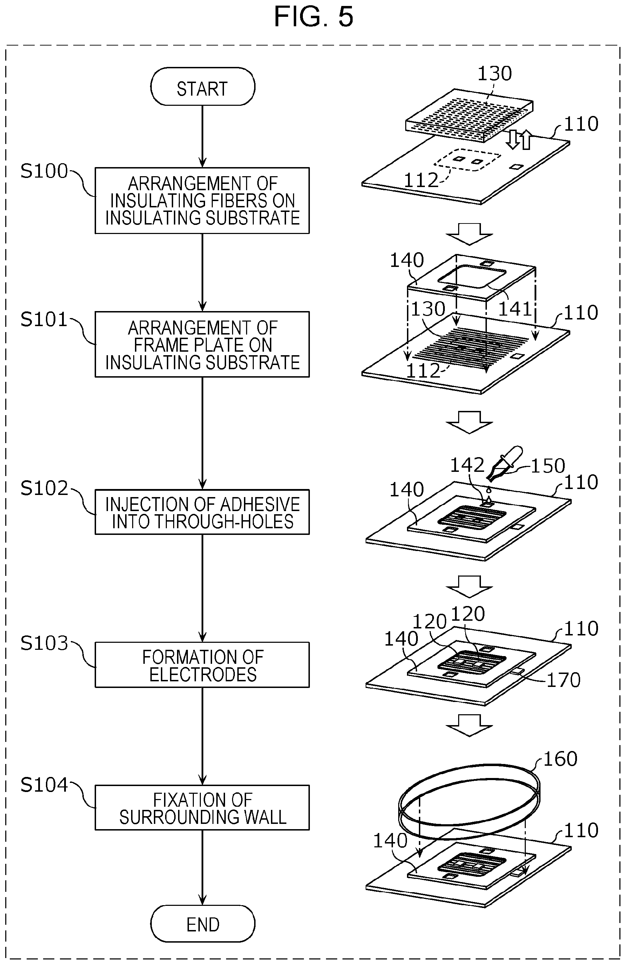

Next, a method for producing the cellular potential measurement substrate 100 that is configured as described above will be described with reference to Fig. 5. Fig. 5 is a flowchart illustrating a method for producing a cellular potential measurement substrate 100 according to Embodiment 1.

First, insulating fibers 130 are disposed on an insulating substrate 110 (S100). Here, for example, aluminum tape or paper tape is prepared in which insulating fibers formed of polymethylglutarimide are formed on a surface of the tape by an electrospinning method. Such aluminum tape or paper tape is pressed against a culture region 112 of the insulating substrate 110 and then peeled off. Thus, the insulating fibers 130 are disposed on the culture region 112.

The insulating substrate 110 can be produced, for example, as follows. First, electrical contacts and wiring lines are formed on a square glass plate having a thickness of 0.7 millimeters. The wiring lines are formed by etching, with a photoresist, an indium tin oxide film having a thickness of 150 nanometers. The surface of the glass plate is then covered with an insulating film formed of a photosensitive acrylic resin. In this step, the electrical contacts and leading end portions of the wiring lines are not covered with the insulating film. Subsequently, the glass substrate is subjected to plasma surface treatment at an RF power of 18 W for two minutes by using a plasma treatment apparatus to produce the insulating substrate 110.

Next, a frame plate 140 is disposed on the insulating substrate 110 such that the position of the culture region 112 of the insulating substrate 110 and the position of a culture hole 141 of the frame plate 140 are aligned with each other (S101). Consequently, portions of the insulating fibers 130 are sandwiched between the frame plate 140 and the insulating substrate 110.

Next, an adhesive 150 is injected into first adhesion through-holes 142 (S102). For example, a silicone resin before curing is dropped in the first adhesion through-holes 142. The insulating substrate 110 is then left to stand for two hours in an oven whose temperature is maintained at 65 degrees Celsius. As a result, the adhesive 150 spreads between the insulating substrate 110 and the frame plate 140, and the frame plate 140 is bonded to the insulating substrate 110.

Subsequently, measurement electrodes 120 and a reference electrode 170 are formed (S103). For example, the measurement electrodes 120 and the reference electrode 170 are formed by covering leading end portions of the wiring lines exposed on the main surface 111 of the insulating substrate 110 with platinum black by plating treatment. Specifically, the leading end portions of the wiring lines are immersed in a plating solution, and a current is allowed to flow at a current density of 20 mA/cm² for two minutes. Thus, the leading end portions of the wiring lines are plated. During plating, the wiring lines function as a cathode. A solution having the composition shown in Table 1 below can be used as the plating solution.

**[Table 1]**

| Composition | Chemical formula | Concentration |
|---|---|---|
| Hexachloroplatinic(IV) acid | H₂PtCl₆·6H₂O | 1% |
| Lead acetate | (CH₃COO)₂Pb·3H₂O | 0.01% |
| Hydrochloric acid | HCI | 0.0025% |

Lastly, a surrounding wall 160 is fixed to the insulating substrate 110 (S104). For example, the surrounding wall 160 is bonded to the insulating substrate 110 with a silicone adhesive.

### [Advantageous effects and the like]

As described above, the cellular potential measurement substrate 100 according to this embodiment includes an insulating substrate 110 having, on a main surface 111 thereof, a culture region 112 for culturing a cell, measurement electrodes 120 disposed in the culture region 112 and configured to measure a potential of the cell, a plurality of insulating fibers 130 arranged on the culture region 112 side by side at intervals in the Y-direction and each extending in the X-direction, a frame plate 140 disposed on the insulating fibers 130 and having a culture hole 141 at a position corresponding to the culture region 112, and an adhesive 150 that has bonded the frame plate 140 to the insulating substrate 110, in which the frame plate 140 further has first adhesion through-holes 142 into which the adhesive 150 has been injected, and the first adhesion through-holes 142 are each disposed in a first region 146 that does not overlap the culture hole 141 when viewed in the X-direction.

With this structure, the first adhesion through-holes 142 are each disposed in the first region 146 that does not overlap the culture hole 141 when viewed in the X-direction. Accordingly, when the adhesive 150 injected in the first adhesion through-holes 142 spreads along the insulating fibers 130, the adhesive 150 can be prevented from reaching the culture hole 141. Therefore, the cellular potential measurement substrate 100 can reduce entering of the adhesive 150 into the culture region 112. As a result, the cellular potential measurement substrate 100 can suppress a decrease in measurement accuracy of the potential due to, for example, covering of the measurement electrodes 120 in the culture region 112 with the adhesive 150. Furthermore, the cellular potential measurement substrate 100 can suppress, for example, inhibition of the cell culture by the adhesive 150 in the culture region 112.

In the cellular potential measurement substrate 100 according to this embodiment, the frame plate 140 has no adhesion through-hole in the second region 147 that overlaps the culture hole 141 when viewed in the X-direction.

This structure can eliminate entering of the adhesive 150 that has been injected into the adhesion through-holes into the culture region 112 in the X-direction and can suppress the phenomenon that the adhesive 150 spreads along the insulating fibers 130 and thereby enters the culture region 112.

The method for producing a cellular potential measurement substrate 100 according to this embodiment includes a step (S100) of arranging, on an insulating substrate 110 having a culture region 112 in which measurement electrodes 120 are disposed, a plurality of insulating fibers 130 side by side at intervals in the Y-direction, the insulating fibers 130 each extending in the X-direction, a step (S101) of disposing, on the insulating substrate 110, a frame plate 140 having a culture hole 141 such that a position of the culture hole 141 and a position of the culture region 112 are aligned with each other, and a step (S102) of injecting an adhesive 150 into first adhesion through-holes 142 formed in first regions 146 of the frame plate 140, the first regions 146 not overlapping the culture hole 141 when viewed in the X-direction.

With this configuration, the frame plate 140 can be disposed on the insulating substrate 110 before the adhesive 150 is injected into the first adhesion through-holes 142, and thus the alignment between the culture hole 141 and the culture region 112 can be facilitated. In addition, since the adhesive 150 is injected into the first adhesion through-holes 142 formed in the first regions 146 of the frame plate 140, the first regions 146 not overlapping the culture hole 141 when viewed in the X-direction, it is possible to reduce entering of the adhesive 150 into the culture region 112.

### (Modification 1 of Embodiment 1)

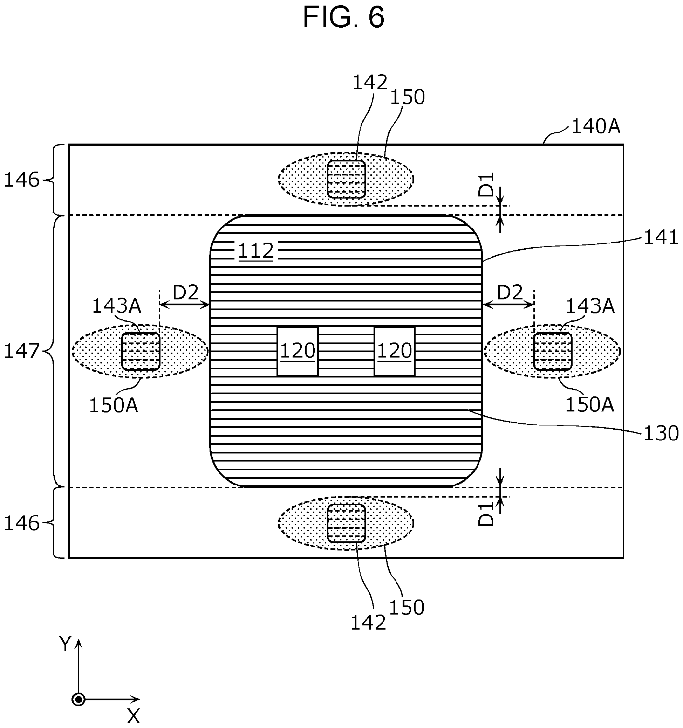

Next, Modification 1 of Embodiment 1 described above will be described. This modification differs from Embodiment 1 in that second adhesion through-holes are disposed in a second region. This modification will be specifically described with reference to Fig. 6 by focusing on points that are different from Embodiment 1.

### [Structure of frame plate]

Fig. 6 is an enlarged plan view of a portion of a frame plate 140A of a cellular potential measurement substrate 100 according to Modification 1 of Embodiment 1. As illustrated in Fig. 6, the frame plate 140A according to this modification has, in addition to a culture hole 141 and first adhesion through-holes 142, second adhesion through-holes 143A arranged to be spaced apart from the culture hole 141 and the first adhesion through-holes 142.

The second adhesion through-holes 143A penetrate the frame plate 140A in the Z-direction and each have a rectangular shape with rounded corners in plan view. An adhesive 150A is injected into the second adhesion through-holes 143A. At least a portion of the second adhesion through-holes 143A is disposed in a second region 147 that overlaps the culture hole 141 when viewed in the X-direction. In this case, a distance D2 between each second adhesion through-hole 143A and the culture hole 141 is larger than a distance D1 between each first adhesion through-hole 142 and the culture hole 141. In addition, the distance D2 is larger than a distance that the adhesive 150A spreads from the second adhesion through-holes 143A in the X-direction. With this structure, the adhesive 150A does not reach the culture hole 141.

In Fig. 6, the distance between two holes is defined by the shortest distance between edges of the two holes. However, the definition of the distance is not limited to this. For example, the distance between two holes may be defined by the linear distance between the centers of the two holes.

### [Advantageous effects and the like]

As described above, in the cellular potential measurement substrate 100 according to this modification, the frame plate 140A further has the second adhesion through-holes 143A into which the adhesive 150A has been injected. At least a portion of the second adhesion through-holes 143A is disposed in the second region 147 that overlaps the culture hole 141 when viewed in the X-direction. The distance D2 between each second adhesion through-hole 143A and the culture hole 141 is larger than the distance D1 between each first adhesion through-hole 142 and the culture hole 141.

With this structure, since the adhesive 150A can be injected into the second adhesion through-holes 143A in addition to the first adhesion through-holes 142, the frame plate 140A can be strongly bonded to the insulating substrate 110 compared with Embodiment 1 described above. In addition, since the distance D2 between the second adhesion through-hole 143A and the culture hole 141 is larger than the distance D1 between the first adhesion through-hole 142 and the culture hole 141, it is possible to suppress the phenomenon that the adhesive 150A injected into the second adhesion through-holes 143A reaches the culture hole 141.

### (Modification 2 of Embodiment 1)

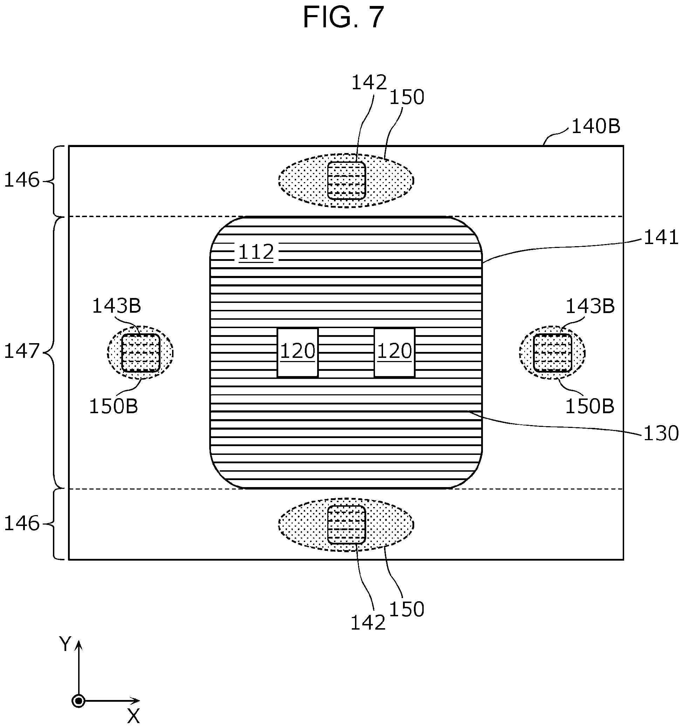

Next, Modification 2 of Embodiment 1 described above will be described. In this modification, second adhesion through-holes are disposed in a second region as in Modification 1 described above, but the amount of adhesive injected into each of the second adhesion through-holes is different from that in Modification 1. This modification will be specifically described with reference to Fig. 7 by focusing on points that are different from Embodiment 1 and Modification 1 of Embodiment 1.

### [Structure of frame plate]

Fig. 7 is an enlarged plan view of a portion of a frame plate 140B of a cellular potential measurement substrate 100 according to Modification 2 of Embodiment 1. As illustrated in Fig. 7, the frame plate 140B according to this modification has, in addition to a culture hole 141 and first adhesion through-holes 142, second adhesion through-holes 143B arranged to be spaced apart from the culture hole 141 and the first adhesion through-holes 142.

The second adhesion through-holes 143B penetrate the frame plate 140B in the Z-direction and each have a rectangular shape with rounded corners in plan view. An adhesive 150B is injected into the second adhesion through-holes 143B. At least a portion of the second adhesion through-holes 143B is disposed in a second region 147 that overlaps the culture hole 141 when viewed in the X-direction. In this case, the amount of adhesive 150B injected into each of the second adhesion through-holes 143B is smaller than the amount of adhesive 150 injected into each of the first adhesion through-holes 142. With this structure, the regions in which the adhesive 150B spreads between the frame plate 140B and the insulating substrate 110 are smaller than the regions in which the adhesive 150 spreads.

### [Advantageous effects and the like]

As described above, in the cellular potential measurement substrate 100 according to this modification, the frame plate 140B further has the second adhesion through-holes 143B into which the adhesive 150B has been injected. At least a portion of the second adhesion through-holes 143B is disposed in the second region 147 that overlaps the culture hole 141 when viewed in the X-direction. The amount of adhesive 150B injected into each of the second adhesion through-holes 143B is smaller than the amount of adhesive 150 injected into each of the first adhesion through-holes 142.

With this structure, since the adhesive 150B can be injected into the second adhesion through-holes 143B in addition to the first adhesion through-holes 142, the frame plate 140B can be strongly bonded to the insulating substrate 110 compared with Embodiment 1 described above. In addition, since the amount of adhesive 150B injected into each of the second adhesion through-holes 143B can be smaller than the amount of adhesive 150 injected into each of the first adhesion through-holes 142, the distance between each second adhesion through-hole 143B and the culture hole 141 need not be larger than the distance between each first adhesion through-hole 142 and the culture hole 141. Accordingly, a reduction in the size of the cellular potential measurement substrate 100 can be realized compared with Modification 1.

### (Embodiment 2)

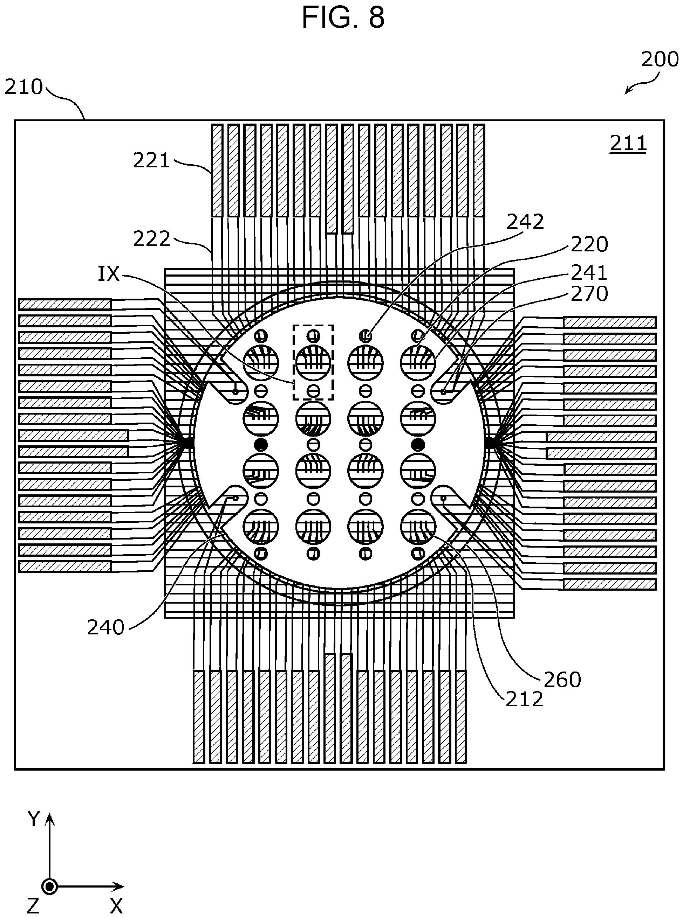

Next, Embodiment 2 will be described. The present embodiment differs mainly from Embodiment 1 described above in that a plurality of culture holes are formed in a frame plate. The present embodiment will be specifically described below with reference to Figs. 8 to 12 by focusing on points that are different from Embodiment 1.

### [Structure of cellular potential measurement substrate]

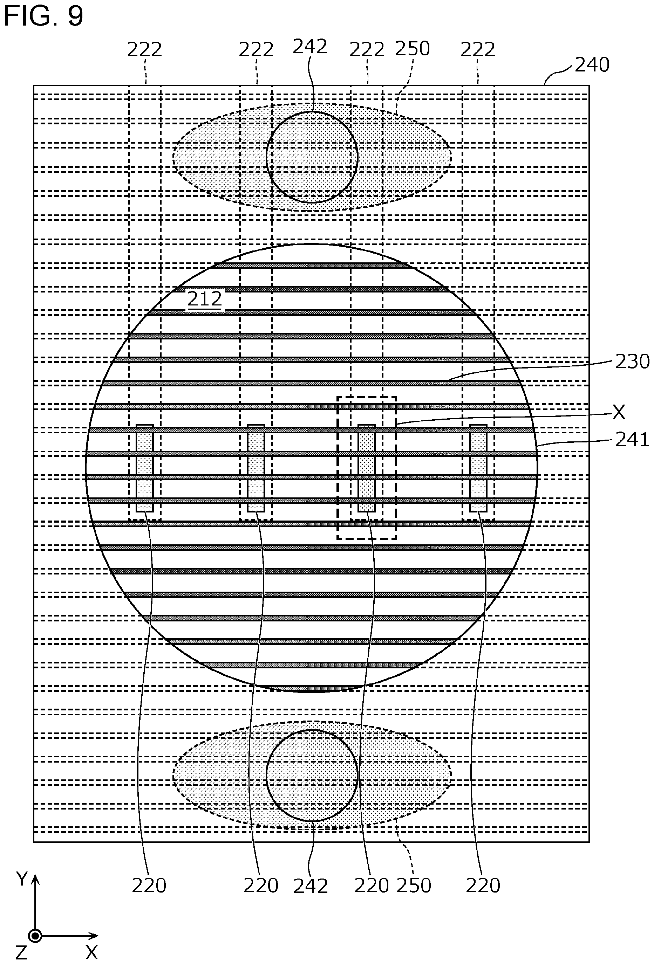

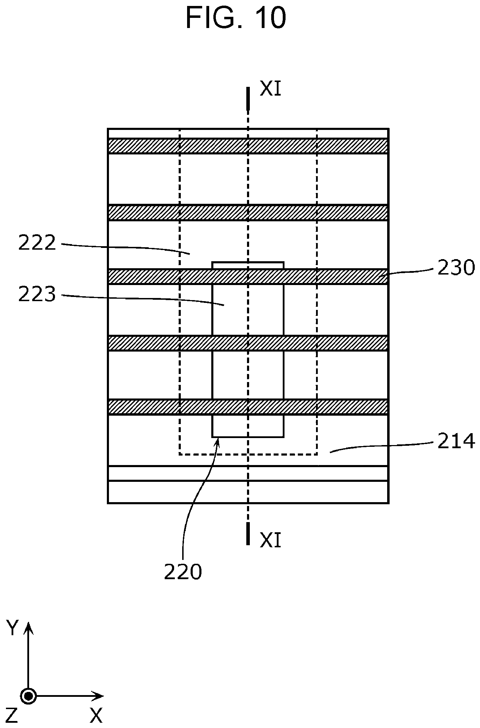

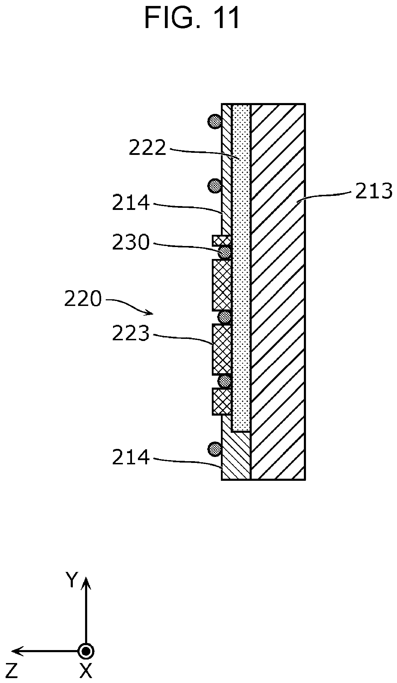

First, the structure of a cellular potential measurement substrate 200 according to the present embodiment will be described with reference to Figs. 8 to 11. Fig. 8 is a plan view of a cellular potential measurement substrate 200 according to Embodiment 2. Fig. 9 is an enlarged plan view of the cellular potential measurement substrate 200 according to Embodiment 2 and is specifically an enlarged view of region IX in Fig. 8. Fig. 10 is a plan view of a measurement electrode 220 in Embodiment 2 and is an enlarged view of region X in Fig. 9. Fig. 11 is a sectional view of the measurement electrode 220 in Embodiment 2 and is a sectional view taken along cutting line XI in Fig. 10.

The cellular potential measurement substrate 200 is a plate-like assembly configured to measure the electric potential of a cell. As illustrated in Figs. 8 and 9, the cellular potential measurement substrate 200 includes an insulating substrate 210, measurement electrodes 220, insulating fibers 230, a frame plate 240, an adhesive 250, a surrounding wall 260, and reference electrodes 270. Each of the components of the cellular potential measurement substrate 200 will be described in detail below.

The insulating substrate 210 has, on a main surface 211, a plurality of culture regions 212 for culturing cells. In this embodiment, the insulating substrate 210 has a size of 50 millimeters × 50 millimeters, the number of plurality of culture regions 212 is 16, and the shape of each of the plurality of culture regions 212 is a circular in plan view. However, the structure in this embodiment is not limited to this.

As illustrated in Figs. 8 and 9, four measurement electrodes 220 are disposed in each of the culture regions 212. As illustrated in Figs. 10 and 11, each of the measurement electrodes 220 is formed, by using platinum black 223, on a leading end portion of a wiring line 222 formed on a glass plate 213, the leading end portion not being covered with an insulating film 214. The measurement electrodes 220 are individually connected through the wiring lines 222 to electrical contacts 221 formed along edges of the insulating substrate 210. The electrical contacts 221 are used to connect to a potential measurement device, for example.

The insulating fibers 230 are arranged on the culture regions 212 side by side at intervals in the Y-direction and each extend in the X-direction, as in Embodiment 1. Portions of the insulating fibers 230 are sandwiched between the frame plate 240 and the insulating substrate 210.

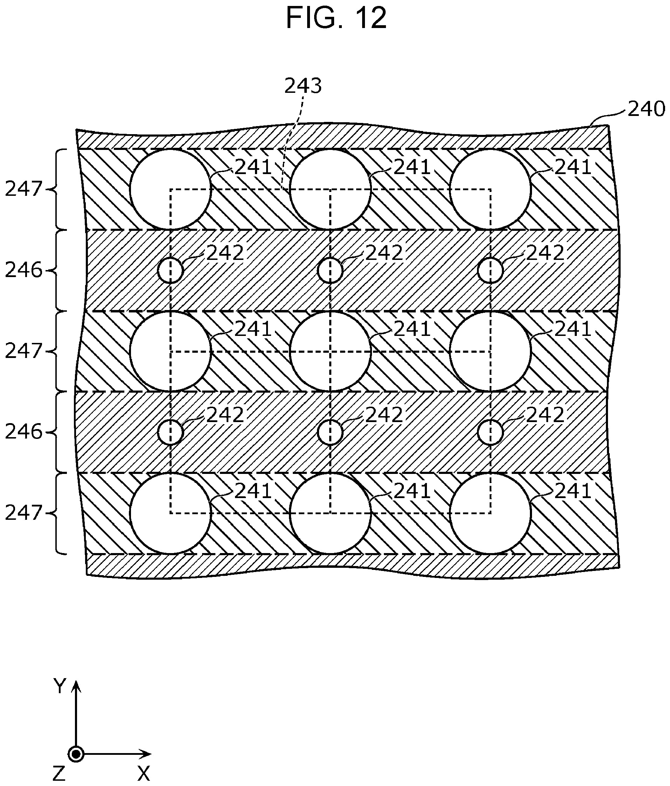

The frame plate 240 is disposed on the insulating fibers 230 as in Embodiment 1. In this embodiment, the frame plate 240 has a plurality of culture holes 241 and a plurality of first adhesion through-holes 242 that are arranged to be spaced apart from each other. The detailed structure of the frame plate 240 will be described later with reference to Fig. 12.

The adhesive 250 bonds the frame plate 240 to the insulating substrate 210 as in Embodiment 1. In the production process of the cellular potential measurement substrate 200, the adhesive 250 is injected into the first adhesion through-holes 242 and spreads in the X-direction mainly along the insulating fibers 230 between the frame plate 240 and insulating substrate 210, as illustrated in Fig. 9.

The surrounding wall 260 is a wall member that surrounds the plurality of culture regions 212 and the frame plate 240. In this embodiment, the surrounding wall 260 is a ring-shaped glass member having an inner diameter of 22 millimeters, an outer diameter of 25 millimeters, and a height of 10 millimeters but is not limited to this.

The reference electrodes 270 are disposed in regions outside the culture regions 212, the regions being included in the region surrounded by the surrounding wall 260 on the main surface 211 of the insulating substrate 210. In this embodiment, the number of reference electrodes 270 is four. Each of the reference electrodes 270 is formed, by using platinum black 223, on a leading end portion of a wiring line 222 formed on the glass plate 213, the leading end portion not being covered with the insulating film 214, as in the measurement electrodes 220.

### [Structure of frame plate]

Next, the structure of the frame plate 240 will be specifically described with reference to Fig. 12. Fig. 12 is an enlarged plan view of the frame plate 240 of the cellular potential measurement substrate 200 according to Embodiment 2. In Fig. 12, first regions 246 and second regions 247 are indicated by hatched patterns having different widths and extending different directions.

The frame plate 240 has a plurality of culture holes 241 and a plurality of first adhesion through-holes 242, as described above.

Each of the plurality of culture holes 241 penetrates the frame plate 240 in the Z-direction and is disposed at a position corresponding to one of the plurality of culture regions 212. In this embodiment, each of the plurality of culture holes 241 has a circular shape in plan view. The plurality of culture holes 241 are arranged in a pattern of a square grid 243 having sides extending in the X-direction and sides extending in the Y-direction. The square grid is one of grids in the two-dimensional Euclidean space. In the square grid, a square can be formed when points that are closest in different directions are sequentially connected.

Each of the plurality of first adhesion through-holes 242 penetrates the frame plate 240 in the Z-direction and has a circular shape in plan view. The plurality of first adhesion through-holes 242 are disposed in the first regions 246 that do not overlap any of the plurality of culture holes 241 when viewed in the X-direction. That is, the plurality of first adhesion through-holes 242 are disposed outside the second regions 247 that overlap any of the plurality of culture holes 241 when viewed in the X-direction. In this case, the plurality of first adhesion through-holes 242 are arranged on the sides of the square grid 243, the sides extending in the Y-direction.

In this embodiment, since each of the plurality of culture holes 241 has a circular shape, the first regions 246 are divided from the second regions 247 by straight lines that are in contact with the outer edges of the plurality of culture holes 241 and that extend in the X-direction.

Note that the frame plate 240 has no adhesion through-hole in the second regions 247. That is, adhesion through-holes are present only in the first regions 246 and are not present in the second regions 247.

### [Advantageous effects and the like]

As described above, in the cellular potential measurement substrate 200 according to this embodiment, the frame plate 240 has a plurality of culture holes 241 that are arranged in a pattern of a square grid 243 having sides extending in the X-direction and sides extending in the Y-direction, and a plurality of first adhesion through-holes 242 disposed in the first regions 246, in which the first regions 246 are regions that do not overlap any of the plurality of culture holes 241 when viewed in the X-direction.

With this structure, the plurality of culture holes 241 can be arranged in a pattern of a square grid 243, and the plurality of culture regions 212 can be efficiently disposed on the insulating substrate 210. As a result, a reduction in the size of the cellular potential measurement substrate 200 can be realized. Furthermore, the plurality of first adhesion through-holes 242 can be disposed in the first regions 246 that do not overlap any of the plurality of culture holes 241 when viewed in the X-direction. Accordingly, when the adhesive 250 injected in the first adhesion through-holes 242 spreads along the insulating fibers 230 in the X-direction, the adhesive 250 can be prevented from reaching the plurality of culture holes 241. Consequently, in the cellular potential measurement substrate 200, entering of the adhesive 250 into the plurality of culture regions 212 can be reduced while a reduction in the size of the cellular potential measurement substrate 200 having the plurality of culture regions 212 is realized.

In the cellular potential measurement substrate 200 according to this embodiment, the plurality of first adhesion through-holes 242 are arranged on the sides of the square grid 243, the sides extending in the Y-direction.

With this structure, the plurality of culture holes 241 and the plurality of first adhesion through-holes 242 can be efficiently disposed in the frame plate 240. Entering of the adhesive 250 into the plurality of culture regions 212 can be reduced while a reduction in the size of the cellular potential measurement substrate 200 is realized.

### (Modification 1 of Embodiment 2)

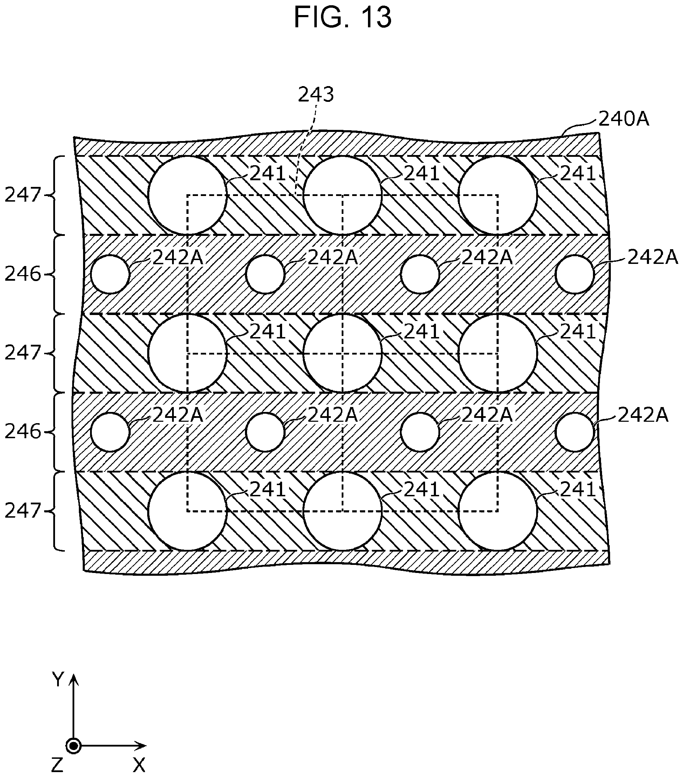

Next, Modification 1 of Embodiment 2 described above will be described. This modification differs from Embodiment 2 in arrangement of a plurality of first adhesion through-holes. This modification will be specifically described with reference to Fig. 13 by focusing on points that are different from Embodiment 2.

### [Structure of frame plate]

Fig. 13 is an enlarged plan view of a frame plate 240A of a cellular potential measurement substrate 200 according to Modification 1 of Embodiment 2. The frame plate 240A according to this modification has a plurality of culture holes 241 and a plurality of first adhesion through-holes 242A.

The plurality of first adhesion through-holes 242A are disposed in first regions 246 that do not overlap any of the plurality of culture holes 241 when viewed in the X-direction, as in Embodiment 2 described above. In this modification, the plurality of first adhesion through-holes 242A are arranged on diagonal lines of a square grid 243.

### [Advantageous effects and the like]

As described above, in the cellular potential measurement substrate 200 according to this modification, the plurality of first adhesion through-holes 242A are arranged on diagonal lines of the square grid 243.

With this structure, the plurality of culture holes 241 and the plurality of first adhesion through-holes 242A can be efficiently disposed in the frame plate 240A. Entering of the adhesive 250 into the plurality of culture regions 212 can be reduced while a reduction in the size of the cellular potential measurement substrate 200 is realized.

### (Modification 2 of Embodiment 2)

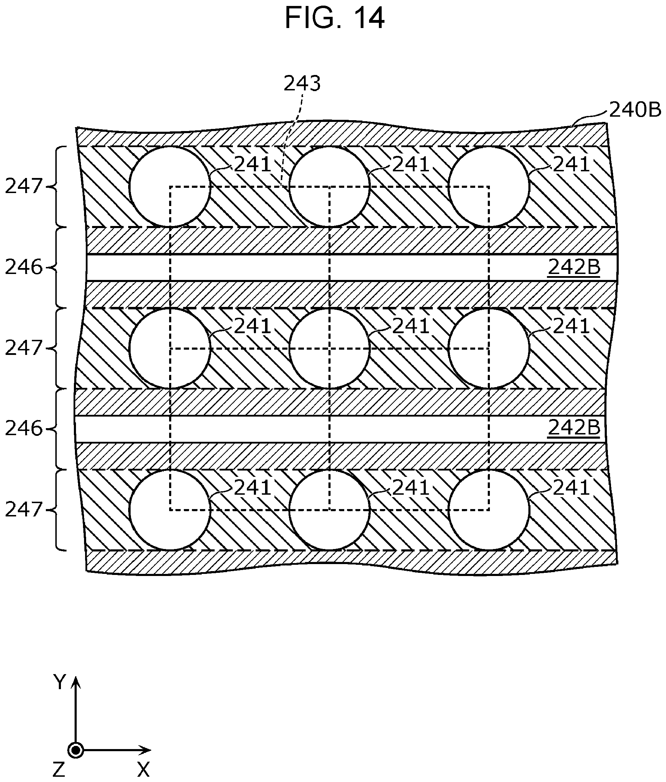

Next, Modification 2 of Embodiment 2 described above will be described. This modification differs from Embodiment 2 in shape of a plurality of first adhesion through-holes. This modification will be specifically described with reference to Fig. 14 by focusing on points that are different from Embodiment 2.

### [Structure of frame plate]

Fig. 14 is an enlarged plan view of a frame plate 240B of a cellular potential measurement substrate 200 according to Modification 2 of Embodiment 2. The frame plate 240B according to this modification has a plurality of culture holes 241 and a plurality of first adhesion through-holes 242B. As illustrated in Fig. 14, each of the plurality of first adhesion through-holes 242B is a long hole extending in the X-direction. Each of the plurality of first adhesion through-holes 242B extends along, among the plurality of culture holes 241, greater than or equal to two culture holes arranged side by side in the X-direction. That is, each of the plurality of first adhesion through-holes 242B corresponds to greater than or equal to two culture holes arranged side by side in the X-direction.

### [Advantageous effects and the like]

As described above, in the cellular potential measurement substrate 200 according to this modification, each of the plurality of first adhesion through-holes 242B is a long hole extending in the X-direction.

With this structure, since the total area of the plurality of first adhesion through-holes 242B can be made larger than the total area of the plurality of first adhesion through-holes 242 in Embodiment 2, the amount of adhesive 250 can be increased. As a result, adhesive force of the frame plate 240B to the insulating substrate 210 can be increased. Furthermore, the number of plurality of first adhesion through-holes 242B can be made smaller than the number of plurality of first adhesion through-holes 242 in Embodiment 2. Therefore, the step of injecting the adhesive 250 can be simplified in the production of the cellular potential measurement substrate 200.

### (Modification 3 of Embodiment 2)

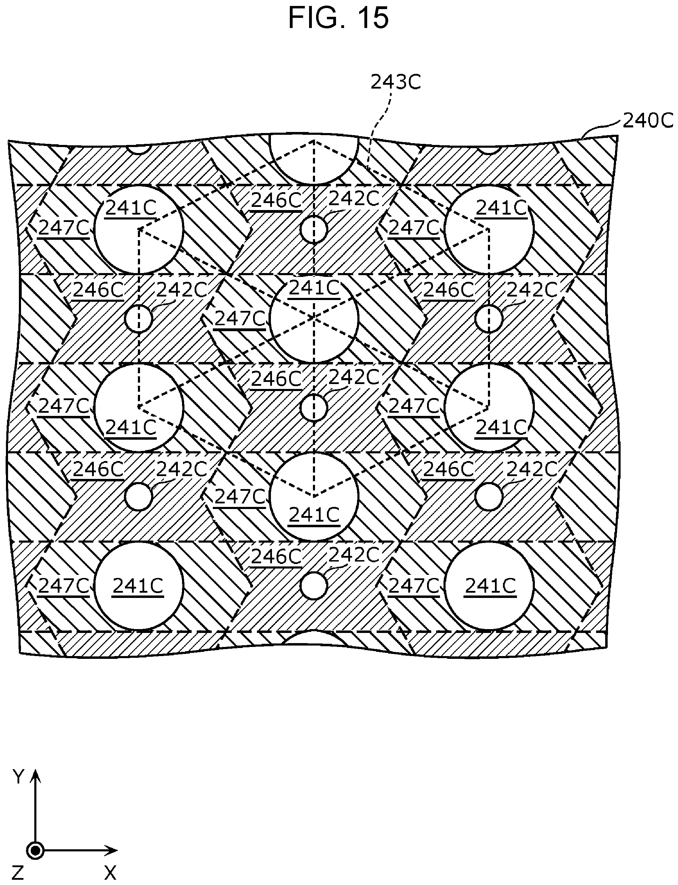

Next, Modification 3 of Embodiment 2 described above will be described. This modification differs from Embodiment 2 in arrangement of a plurality of culture holes and accordingly differs from Embodiment 2 in arrangement of a plurality of first adhesion through-holes. This modification will be specifically described with reference to Fig. 15 by focusing on points that are different from Embodiment 2.

### [Structure of frame plate]

Fig. 15 is an enlarged plan view of a frame plate 240C of a cellular potential measurement substrate 200 according to Modification 3 of Embodiment 2. The frame plate 240C according to this modification has a plurality of culture holes 241C and a plurality of first adhesion through-holes 242C.

The plurality of culture holes 241C are arranged in a pattern of a hexagonal grid 243C having sides in the Y-direction. The hexagonal grid is one of grids in the two-dimensional Euclidean space. In the hexagonal grid, a regular hexagon and a regular triangle can be formed when points that are closest in different directions are sequentially connected. The hexagonal grid may also be referred to as a regular triangular grid.

The plurality of first adhesion through-holes 242C are disposed in first regions 246C that do not overlap the closest culture holes among the plurality of culture holes 241C, when viewed in the X-direction. That is, the plurality of first adhesion through-holes 242C are disposed outside second regions 247C that overlap the closest culture holes among the plurality of culture holes 241C, when viewed in the X-direction. In this case, the plurality of first adhesion through-holes 242C are arranged on the sides of the hexagonal grid 243C, the sides extending in the Y-direction.

### [Advantageous effects and the like]

As described above, in the cellular potential measurement substrate 200 according to this modification, the frame plate 240C has the plurality of culture holes 241C arranged on a main surface 211 in the pattern of the hexagonal grid 243C having sides extending in the Y-direction perpendicular to the X-direction and the plurality of first adhesion through-holes 242C disposed in the first regions 246C. The first regions 246C are regions that do not overlap the closest culture holes among the plurality of culture holes 241C, when viewed in the X-direction.

With this structure, the plurality of culture holes 241C can be arranged in the pattern of the hexagonal grid 243C, and thus the plurality of culture regions 212 can be efficiently disposed on the insulating substrate 210. As a result, a reduction in the size of the cellular potential measurement substrate 200 can be realized. Furthermore, the plurality of first adhesion through-holes 242C can be disposed in the first regions 246C that do not overlap the closest culture holes among the plurality of culture holes 241C, when viewed in the X-direction. Accordingly, when the adhesive 250 injected into the plurality of first adhesion through-holes 242C spreads along the insulating fibers 230, the adhesive 250 can be prevented from reaching the plurality of culture holes 241C. Consequently, in the cellular potential measurement substrate 200, entering of the adhesive 250 into the plurality of culture regions 212 can be reduced while the reduction in the size of the cellular potential measurement substrate 200 having the plurality of culture regions 212 is realized.

In the cellular potential measurement substrate 200 according to this embodiment, the plurality of first adhesion through-holes 242C are arranged on the sides of the hexagonal grid 243C, the sides extending in the Y-direction.

With this structure, the plurality of culture holes 241C and the plurality of first adhesion through-holes 242C can be efficiently disposed in the frame plate 240C. Entering of the adhesive 250 into the plurality of culture regions 212 can be reduced while a reduction in the size of the cellular potential measurement substrate 200 is realized.

### (Other embodiments)

Cellular potential measurement substrates and methods for producing the substrates according to one or a plurality of aspects of the present disclosure have been described on the basis of embodiments. However, the present disclosure is not limited to these embodiments. Modified examples obtained by making various modifications to the above-described embodiments by those skilled in the art and other embodiments formed by combining components of different embodiments and modifications may also be included in the scope of one or a plurality of aspects of the present disclosure so long as the modified examples and embodiments do not depart from the spirit and scope of the present disclosure.

For example, in each of the embodiments, each of the adhesion through-holes has a circular shape in plan view. However, the shape of the adhesion through-hole is not limited thereto. For example, the adhesion through-hole may have a triangular shape, a rectangular shape, or an elliptical shape.

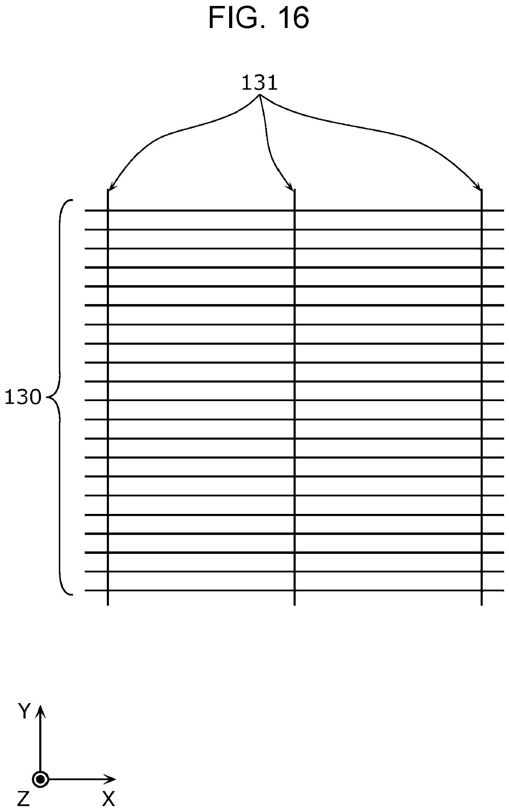

In each of the embodiments, the cellular potential measurement substrates 100 and 200 respectively include insulating fibers 130 and 230 extending in the X-direction. However, the structure is not limited to this. For example, the cellular potential measurement substrate 100 may include, in addition to the insulating fibers 130, insulating fibers that extend in a direction crossing the X-direction or non-oriented insulating fibers. For example, as illustrated in Fig. 16, the cellular potential measurement substrate 100 may include insulating fibers 131 extending the Y-direction in addition to insulating fibers 130 extending in the X-direction. In this case, when the ratio of the number of insulating fibers 130 to the number of insulating fibers 131 is an appropriate ratio (for example, 10:1), cells can be effectively cultured while twisting the insulating fibers 130 is suppressed.

In Embodiment 1 described above, the number of first adhesion through-holes 142 is two but is not limited to this. The number of first adhesion through-holes 142 may be one or greater than or equal to three.

In the embodiments described above, the surrounding walls 160 and 260 are ring-shaped glass members. The shape and the material of the surrounding walls 160 and 260 are not limited to the above. For example, the surrounding walls 160 and 260 may have a rectangular tubular shape and may be resin members.

The numbers of measurement electrodes 120 and 220 in the embodiments are examples, and no particular limitation is imposed on the numbers. For example, the number of measurement electrodes 120 and the number of measurement electrodes 220 may each be one.

In the embodiments described above, the measurement electrodes 120 and 220 are used to measure the electric potentials of cells. However, the use of the measurement electrodes 120 and 220 is not limited to this. For example, the measurement electrodes 120 and 220 may be used to apply electric pulses to cells.

The order of the processes in the production method in Fig. 5 according to Embodiment 1 is a non-limiting example. For example, the measurement electrodes 120 and the reference electrode 170 may be formed after the surrounding wall 160 is fixed. Alternatively, exposed portions of the wiring lines may be used as electrodes as they are. In such a case, the step of forming the measurement electrodes 120 and the reference electrode 170 may be omitted.

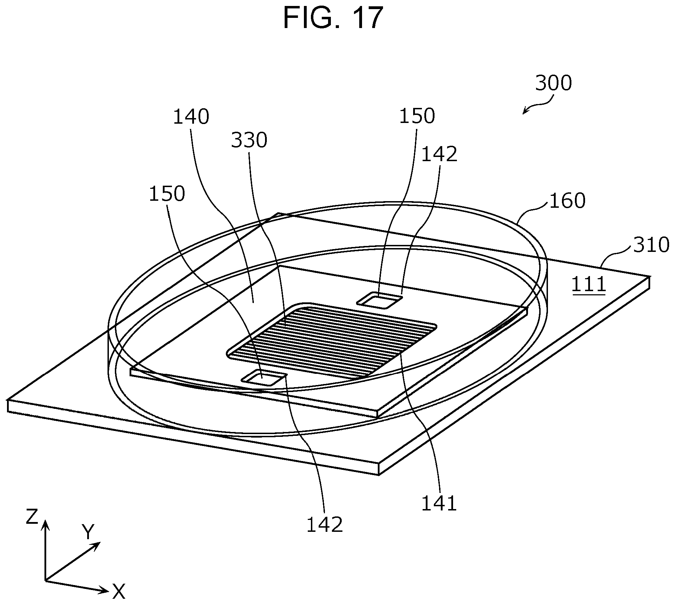

In the embodiments and modifications described above, electrodes are disposed on a substrate. However, the structure is not limited to this. That is, the present disclosure may be applied to a cell culture substrate that does not require measurement of an electric potential. Fig. 17 is a perspective view of a cell culture substrate 300 according to another embodiment. As illustrated in Fig. 17, in the cell culture substrate 300, with respect to the cellular potential measurement substrate 100 illustrated in Fig. 1, the measurement electrodes 120 and the reference electrode 170 are removed, and the insulating substrate 110 and the insulating fibers 130 are replaced by a substrate 310 and fibers 330, respectively.

Specifically, the cell culture substrate 300 includes a substrate 310 having, on a main surface thereof, a culture region 112 for culturing a cell, a plurality of fibers 330 which are disposed in the culture region 110, which are arranged on the culture region 110 side by side at intervals in the Y-direction, and each of which extends on the main surface in the X-direction perpendicular to the Y-direction, a frame plate 140 disposed on the plurality of fibers 330 and having a culture hole 141 at a position corresponding to the culture region 110, and an adhesive 150 that has bonded the frame plate 140 to the substrate 310. The frame plate 140 further has first adhesion through-holes 142 into which the adhesive 150 has been injected, and the first adhesion through-holes 142 are disposed in a first region 146 that does not overlap the culture hole 141 when viewed in the X-direction.

With this structure, the first adhesion through-holes 142 are each disposed in the first region 146 that does not overlap the culture hole 141 when viewed in the X-direction. Accordingly, when the adhesive 150 injected in the first adhesion through-holes 142 spreads along the fibers 330, the adhesive 150 can be prevented from reaching the culture hole 141. Therefore, the cell culture substrate 300 can reduce entering of the adhesive 150 into the culture region 112. As a result, the cell culture substrate 300 can suppress inhibition of the cell culture by the adhesive 150 in the culture region 112.

Here, the cell culture substrate 300 is a modification of the cellular potential measurement substrate 100 according to Embodiment 1 but is not limited to this. For example, the cell culture substrate may be a modification of any of the cellular potential measurement substrates according to the modifications of Embodiment 1, Embodiment 2, and the modifications of Embodiment 2.

### Reference Signs List

100, 200 cellular potential measurement substrate
110, 210 insulating substrate
111, 211 main surface
112, 212 culture region
120, 220 measurement electrode
130, 131, 230 insulating fiber
140, 140A, 140B, 240, 240A, 240B, 240C frame plate
141, 241, 241C culture hole
142, 242, 242A, 242B, 242C first adhesion through-hole
143A, 143B second adhesion through-hole
146, 246, 246C first region
147, 247, 247C second region
150, 150A, 150B, 250 adhesive
160, 260 surrounding wall
170, 270 reference electrode
213 glass plate
214 insulating film
221 electrical contact
222 wiring line
223 platinum black
243 square grid
243C hexagonal grid
300 cell culture substrate
310 substrate
330 fiber

## Claims

1. A cellular potential measurement substrate comprising:
an insulating substrate having, on a main surface thereof, a culture region for culturing a cell;
a measurement electrode disposed in the culture region and configured to measure a potential of the cell;
a plurality of insulating fibers arranged on the culture region side by side at intervals in a second direction and each extending on the main surface in a first direction perpendicular to the second direction;
a frame plate disposed on the plurality of insulating fibers and having a culture hole at a position corresponding to the culture region; and
an adhesive that has bonded the frame plate to the insulating substrate,
wherein the frame plate further has a first adhesion through-hole into which the adhesive has been injected, and
the first adhesion through-hole is disposed in a first region that does not overlap the culture hole when viewed in the first direction.

2. The cellular potential measurement substrate according to Claim 1,
wherein the frame plate has no adhesion through-hole in a second region that overlaps the culture hole when viewed in the first direction.

3. The cellular potential measurement substrate according to Claim 1,
wherein the frame plate further has a second adhesion through-hole into which the adhesive has been injected,
at least a portion of the second adhesion through-hole is disposed in a second region that overlaps the culture hole when viewed in the first direction, and
a distance between the second adhesion through-hole and the culture hole is larger than a distance between the first adhesion through-hole and the culture hole.

4. The cellular potential measurement substrate according to Claim 1,
wherein the frame plate further has a second adhesion through-hole into which the adhesive has been injected,
at least a portion of the second adhesion through-hole is disposed in a second region that overlaps the culture hole when viewed in the first direction, and
an amount of adhesive injected into the second adhesion through-hole is smaller than an amount of adhesive injected into the first adhesion through-hole.

5. The cellular potential measurement substrate according to any one of Claims 1 to 4,
wherein the frame plate has a plurality of culture holes including the culture hole and a plurality of first adhesion through-holes including the first adhesion through-hole,
the plurality of culture holes are arranged in a pattern of a square grid having sides extending in the first direction and sides extending in the second direction,
the plurality of first adhesion through-holes are disposed in the first region, and
the first region is a region that does not overlap any of the plurality of culture holes when viewed in the first direction.

6. The cellular potential measurement substrate according to Claim 5,
wherein the plurality of first adhesion through-holes are arranged on the sides of the square grid, the sides extending in the second direction.

7. The cellular potential measurement substrate according to Claim 5,
wherein the plurality of first adhesion through-holes are arranged on diagonal lines of the square grid.

8. The cellular potential measurement substrate according to Claim 5,
wherein each of the plurality of first adhesion through-holes is a long hole extending in the first direction.

9. The cellular potential measurement substrate according to Claim 1,
wherein the frame plate has a plurality of culture holes including the culture hole and a plurality of first adhesion through-holes including the first adhesion through-hole,
the plurality of culture holes are arranged on the main surface in a pattern of a hexagonal grid having sides extending in the second direction,
the plurality of first adhesion through-holes are disposed in the first region, and
the first region is a region that does not overlap a closest culture hole among the plurality of culture holes when viewed in the first direction.

10. The cellular potential measurement substrate according to Claim 9,
wherein the plurality of first adhesion through-holes are arranged on the sides of the hexagonal grid, the sides extending in the second direction.

11. A method for producing a cellular potential measurement substrate, comprising:
a step of arranging, on an insulating substrate having a culture region in which a measurement electrode is disposed, a plurality of insulating fibers side by side at intervals in a second direction, the insulating fibers each extending on the main surface in a first direction perpendicular to the second direction;
a step of disposing, on the insulating substrate, a frame plate having a culture hole such that a position of the culture hole and a position of the culture region are aligned with each other; and
a step of injecting an adhesive into a first adhesion through-hole formed in a first region of the frame plate, the first region not overlapping the culture hole when viewed in the first direction.

12. A cell culture substrate comprising:
a substrate having, on a main surface thereof, a culture region for culturing a cell;
a plurality of fibers arranged on the culture region side by side at intervals in a second direction and each extending on the main surface in a first direction perpendicular to the second direction;
a frame plate disposed on the plurality of fibers and having a culture hole at a position corresponding to the culture region; and
an adhesive that has bonded the frame plate to the substrate,
wherein the frame plate further has a first adhesion through-hole into which the adhesive has been injected, and
the first adhesion through-hole is disposed in a first region that does not overlap the culture hole when viewed in the first direction.
